# EUROPEAN PATENT SPECIFICATION

(11) **EP 4 371 596 B1**
(45) Date of publication and mention of the grant of the patent: **09.09.2026**
(21) Application number: 21949719.5
(22) Date of filing: 16.07.2021
(51) Int. Cl.: A61M 16/00

(54) **CARDIOGENIC INTERFERENCE IDENTIFICATION METHOD FOR MEDICAL VENTILATION DEVICE, AND MEDICAL VENTILATION DEVICE**
VERFAHREN ZUR IDENTIFIZIERUNG KARDIOGENER INTERFERENZEN FÜR MEDIZINISCHE BEATMUNGSVORRICHTUNG UND MEDIZINISCHE BEATMUNGSVORRICHTUNG
PROCÉDÉ D'IDENTIFICATION D'INTERFÉRENCE CARDIOGÈNE POUR DISPOSITIF DE VENTILATION MÉDICALE, ET DISPOSITIF DE VENTILATION MÉDICALE

(43) Date of publication of application: 22.05.2024
(73) Proprietor: Shenzhen Mindray Bio-Medical Electronics Co., Ltd., Shenzhen, Guangdong 518057 (CN)
(72) Inventor: ZHU, Feng, Shenzhen, Guangdong 518057 (CN); LIU, Jinglei, Shenzhen, Guangdong 518057 (CN); YAO, Pulin, Shenzhen, Guangdong 518057 (CN); ZHOU, Xiaoyong, Shenzhen, Guangdong 518057 (CN)
(74) Representative: KIPA AB
(86) International application number: PCT/CN2021/106785
(87) International publication number: WO 2023/283935

(56) References cited:
- EP-A1- 1 718 356
- EP-A1- 3 115 075
- WO-A1-2013/084159
- CN-A- 103 619 390
- US-A1- 2012 136 222
- US-A1- 2013 025 597
- US-A1- 2013 025 597
- US-B1- 6 186 956

## Description

### TECHNICAL FIELD

The disclosure relates to a technical field of medical devices, and more particularly to an identification method of cardiogenic interference for a medical ventilation device, and a medical ventilation device.

### BACKGROUND

Medical ventilation devices with an inhalation trigger function improve human-machine synchronism of an autonomous respiration process of a ventilated object. Inhalation trigger means when the ventilated object takes an inhalation action, an inspiratory effort of the ventilated object is sensed by the medical ventilation device and then the ventilation device begins to supply gas. Factors, such as a gas leakage of pipeline, a vibration of pipeline, cardiogenic interference, and the likes, may cause false inhalation trigger of the medical ventilation device. False inhalation trigger, on one hand, may result in excessive ventilation of the ventilated object, causing lung injury and respiratory alkalosis. On the other hand, the false inhalation trigger may be considered that a real inhalation trigger of the ventilated object, which causes a clinical misdiagnosis.

In order to improve a reliability of the medical ventilation device, the current medical ventilation device usually has a leakage compensation function, which avoids the false trigger, which is triggered by the gas leakage, by automatically adapting to different leakages. However, for the false trigger caused by the cardiogenic interference, it can only be prevented by manually adjusting a sensitivity of the inhalation trigger. While the premise of this adjustment operation is that medical staff can identify the false trigger caused by the cardiogenic interference in time. Due to the fact that the false trigger caused by the cardiogenic interference is similar to that of the real inhalation trigger of the ventilated object, in most cases, medical staff with overall experience are difficult to identify it.

### SUMMARY

The invention is defined by the appended claims.

A series of simplified concepts are introduced into summary of this disclosure, and are further elaborated in specific embodiments of this disclosure. The summary of this disclosure does not attempt to define key and necessary technical characteristics of the claimed technical solution, nor attempt to determine a protection scope of the claimed technical solution.

A first aspect according to an embodiment of this disclosure provides an identification method of cardiogenic interference for a medical ventilation device, including:
acquiring a first signal and a second signal by the medical ventilation device through monitoring a ventilated object, wherein the first signal includes a flow rate signal;
identifying a fluctuation of the flow rate signal according to the flow rate signal and the second signal; and
acquiring a fluctuation characteristic of the fluctuation, and identifying the cardiogenic interference in the fluctuation based on the fluctuation characteristic of the fluctuation.

A second aspect according to an embodiment of this disclosure provides an identification method of cardiogenic interference for a medical ventilation device, including:
acquiring a first signal and a second signal by the medical ventilation device through monitoring a ventilated object, wherein the first signal includes a pressure signal;
identifying a fluctuation of the pressure signal according to the pressure signal and the second signal; and
acquiring a fluctuation characteristic of the fluctuation, and identifying the cardiogenic interference in the fluctuation based on the fluctuation characteristic of the fluctuation.

A third aspect according to an embodiment of this disclosure provides an identification method of cardiogenic interference for a medical ventilation device, including:
acquiring a first signal and a second signal by the medical ventilation device through monitoring a ventilated object, wherein the first signal and the second signal are two signals which are capable of reflecting a heartbeat signal of the ventilated object;
identifying a fluctuation of the first signal according to the first signal and the second signal; and
acquiring a fluctuation characteristic of the fluctuation, and identifying the cardiogenic interference in the fluctuation based on the fluctuation characteristic of the fluctuation.

A fourth aspect according to an embodiment of this disclosure provides a medical ventilation device, including:
a ventilation unit, which is configured to provide ventilation to a ventilated object;
a measurement unit, which is configured to monitor the ventilated object during the ventilation to the ventilated object, so as to acquire a first signal and a second signal; and
a processor, which is connected with the measurement unit and configured to acquire the first signal and the second signal, wherein the processor is further configured to execute the above-mentioned identification method of cardiogenic interference.

A fifth aspect according to an embodiment of this disclosure provides an identification method of cardiogenic interference for a medical ventilation device, including:
acquiring a first signal and a second signal, wherein the first signal includes a flow rate signal or a pressure signal which is acquired by the medical ventilation device through monitoring a ventilated object; and
identifying a fluctuation of the first signal and further identifying the cardiogenic interference in the fluctuation based on the second signal.

A sixth aspect according to an embodiment of this disclosure provides a medical ventilation device, including:
a ventilation unit, which is configured to provide ventilation to a ventilated object;
a measurement unit, which is configured to monitor the ventilated object during the ventilation to the ventilated object, so as to acquire a first signal; and
a processor, which is connected with the measurement unit and an external device, and configured to acquire the first signal and a second signal which is acquired by the external device through monitoring the ventilated object; wherein the processor is further configured to execute the above-mentioned identification method of cardiogenic interference.

According to the identification method of cardiogenic interference for a medical ventilation device and the medical ventilation device provided by embodiments of this disclosure, the cardiogenic interference is identified based on physiological signals which are acquired by the medical ventilation device itself, without requiring physiological signals of external device, which has obvious clinical use values.

### BRIEF DESCRIPTION OF THE DRAWINGS

In order to provide a clearer explanation of technical solutions in embodiments of this disclosure, a brief introduction is given to the accompanying drawings required in the description of the embodiments. It is evident that the accompanying drawings in the following description are only some embodiments of this disclosure. For ordinary technical personnel in the art, other accompanying drawings can be obtained based on these drawings without any creative effort.
FIG. 1 is a schematic flowchart of an identification method of cardiogenic interference for a medical ventilation device, according to an embodiment of this disclosure.
FIG. 2 is a schematic diagram of a flow rate signal, a pressure signal, and a cross-correlation signal between the flow rate signal and the pressure signal, according to an embodiment of this disclosure.
FIG. 3 is a schematic diagram of a fluctuation signal of a flow rate according to an embodiment of this disclosure.
FIG. 4 is a schematic diagram showing an evaluation of a magnitude of cardiogenic interference according to an embodiment of this disclosure.
FIG. 5 is a schematic diagram showing an adjustment of an inhalation trigger threshold according to an embodiment of this disclosure.
FIG. 6 is a block diagram of a medical ventilation device according to an embodiment of this disclosure.
FIG. 7 is a schematic flowchart of an identification method of cardiogenic interference for a medical ventilation device, according to another embodiment of this disclosure.
FIG. 8 is a schematic flowchart of an identification method of cardiogenic interference for a medical ventilation device according to a further embodiment of this disclosure.
FIG. 9 is a schematic flowchart of an identification method of cardiogenic interference for a medical ventilation device according to a further another embodiment of this disclosure.
FIG. 10 is a schematic diagram of cardiogenic interference identification based on an ECG signal according to an embodiment of this disclosure.

### DESCRIPTION OF THE PREFERRED EMBODIMENTS

In order to make the purpose, technical solution, and advantages of this disclosure more obvious, the following refers to the attached drawings to describe in detail the exemplary embodiments according to this disclosure. Obviously, the described embodiments are only some of the embodiments of this disclosure, not all of them. It should be understood that this disclosure is not limited by the embodiments described here. Based on the embodiments described in this disclosure, all other embodiments obtained by those skilled in the art without creative work, fall within the protection scope of this disclosure.

In the following description, a large number of specific details are provided to provide a more thorough understanding of this disclosure. However, it is evident to those skilled in the art that this disclosure can be implemented without the need for one or more of these details. In other examples, in order to avoid confusion with this disclosure, some well-known technical characteristics in this field are not described.

It should be understood that this disclosure can be implemented in different forms and should not be limited to the embodiments proposed here. On the contrary, providing these embodiments make the disclosure thorough and complete, and fully convey the scope of this disclosure to those skilled in the art.

The purpose of the terminology used here is only to describe specific embodiments and is not a limitation of this disclosure. When used here, the singular forms of "an", "a", and "the/said" are also intended to include the plural form, unless the context clearly indicates another way. It should also be understood that the terms, such as "including", "and/or", "comprising", when used in this disclosure, determine the presence of the characteristics, integers, steps, operations, components, and/or elements, but do not exclude the presence or addition of one or more other characteristics, integers, steps, operations, components, and/or elements. When used here, the term "and/or" includes any and all combinations of related listed items.

In order to fully understand this disclosure, a detailed structure is provided in the following description to explain the technical solution proposed in this disclosure. The optional embodiments of this disclosure are described in detail below, however, in addition to these detailed description, this disclosure may also have other embodiments.

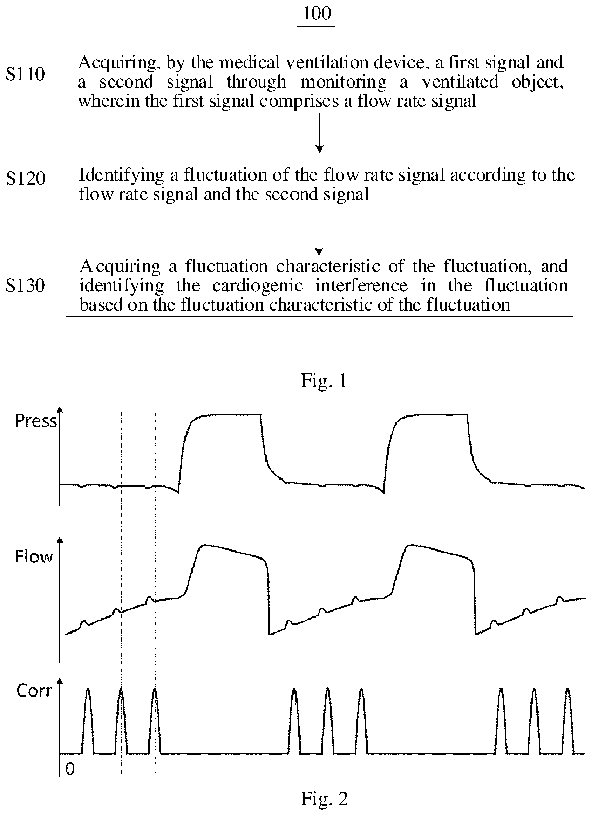

Below, an identification method of cardiogenic interference for a medical ventilation device according to an embodiment of this disclosure is described with reference to FIG. 1. FIG. 1 is a schematic flowchart of an identification method of cardiogenic interference 100 according to an embodiment of this disclosure.

As shown in FIG. 1, the identification method of cardiogenic interference 100 according to an embodiment of this disclosure includes the following steps.

In step S110, a first signal and a second signal are acquired by a medical ventilation device through monitoring a ventilated object, wherein the first signal includes a flow rate signal.

In step S120, a fluctuation of the flow rate signal is identified according to the flow rate signal and the second signal.

In step S130, a fluctuation characteristic of the fluctuation is acquired, and the cardiogenic interference in the fluctuation is identified based on the fluctuation characteristic of the fluctuation.

The identification method of cardiogenic interference 100 of the embodiments of this disclosure identifies the cardiogenic interference based on physiological signals which are acquired by the medical ventilation device itself, so as to inhibit a false trigger which is caused by the cardiogenic interference, and is capable of realizing the identification of cardiogenic interference when no external device for monitoring heartbeat information exists, which has an obvious clinical use value.

Specifically, the medical ventilation device may be implemented as a device for providing mechanical ventilation to the ventilated object, and the ventilated object may specifically refer to a patient who needs to breathe by means of a medical ventilation device due to respiratory failure or difficulty in autonomous respiration. When the respiratory system of the ventilated object cannot complete normal autonomous respiration, mechanical ventilation is performed through the medical ventilation device to provide respiratory support. The medical ventilation device according to the embodiments of this disclosure may be implemented as a medical device having a mechanical ventilation function, such as a ventilator and an anesthesia machine.

A respiratory cycle of the mechanical ventilation completed by the medical ventilation device needs to undergo an inhalation trigger, an inhalation process, an inhalation and exhalation handover, and an exhalation process. Wherein, the ventilated object actively inhales gas to cause a pressure drop or a flow rate change in the airway, and the medical ventilation device senses the inhalation action of the ventilated object and starts to supply gas, which is referred to as an inhalation trigger. The higher the sensitivity of the inhalation trigger, the more rapid the medical ventilation device can sense the inspiratory effort of the ventilated object, and the smaller the respiratory work required for the inhalation trigger of the ventilated object. However, the higher the sensitivity of the inhalation trigger function, the greater the possibility of the false trigger of the medical ventilation device. In numerous factors that cause the false trigger, the false trigger caused by the cardiogenic interference is similar to the real inhalation trigger and is difficult to be identified. Regarding to this problem, the identification method of cardiogenic interference 100 of the embodiments of this disclosure identifies the cardiogenic interference based on the first signal and the second signal which are acquired by the medical ventilation device itself through monitoring the ventilated object.

The modes for the inhalation trigger of the medical ventilation device include, but are not limited to, a flow rate trigger mode and a pressure trigger mode. For the flow rate trigger mode, when the medical ventilation device detects that a flow rate in the airway or a change of the flow rate in the airway is greater than a preset threshold for the flow rate trigger, it is determined that the ventilated object triggers an inhalation. For the pressure trigger mode, when the medical ventilation device detects that an airway pressure is lower than a preset threshold for the pressure trigger, it is determined that the ventilated object triggers an inhalation. The identification method of cardiogenic interference 100 in this embodiment can identify the cardiogenic interference in the flow rate signal, and is mainly applicable to the flow rate trigger mode. Compared with the pressure trigger, the flow rate trigger is more sensitive, and the inhalation load of the ventilated object is less. Meanwhile, due to the fact that the flow rate trigger is relatively sensitive, the false trigger is more easily caused by the cardiogenic interference. Cardiogenic interference is interference caused by heartbeat. In a systolic and diastolic process of the heart, due to a volume change of the heart and a position change of the heart, a gradient change of internal airway pressure, as well as an airflow, are generated, resulting in a fluctuation of the flow rate, and further resulting the false trigger. The identification method of cardiogenic interference provided by the embodiment of this disclosure effectively identifies the cardiogenic interference in the flow rate signal, and inhibits the false trigger caused by the cardiogenic interference through adjusting a trigger threshold, so as to avoid problems of excessive ventilation and the likes caused by the false trigger.

In step S110, the first signal and the second signal are signals which are acquired by the medical ventilation device itself through monitoring the ventilated object during a mechanical ventilation process. For example, the first signal and the second signal are capable of reflecting the inspiratory effort of the ventilated object. The first signal includes a flow rate signal, and specifically may be an airway flow rate which is acquired by a flow rate sensor of the medical ventilation device through monitoring the ventilated object. The second signal may be signals other than the flow rate signal, which signals are also monitored by the medical ventilation device.

In one example, due to the systolic and diastolic process of the heart, a pressure change is caused in thoracic cavity, thereby causing a pressure change in the airway, that is, the pressure signal can significantly reflect the cardiogenic interference. Therefore, the second signal may be a pressure signal measured by the medical ventilation device, and may specifically be a pressure signal reflecting the inspiratory effort of the ventilated object. Exemplarily, the pressure signal which reflects the inspiratory effort of the ventilated object includes at least one of an intrapulmonary pressure signal, an airway pressure signal, an esophageal pressure signal, a transdiaphragmatic pressure signal, an intragastric pressure signal, and a carina pressure signal. Exemplarily, the pressure signal may be measured by a pressure sensor of the medical ventilation device.

Alternatively, the second signal may also be a respiratory system signal which reflects the inspiratory effort of the ventilated object and is acquired by the medical ventilation device through monitoring the ventilated object in the mechanical ventilation process. Exemplarily, the respiratory system signal which reflects the inspiratory effort of the ventilated object includes at least one of a lung ultrasound imaging signal, a diaphragm ultrasound imaging signal, an impedance imaging signal, and a diaphragm electrical signal, wherein the impedance imaging signal includes, but is not limited to, a chest impedance imaging signal.

Alternatively, the second signal may also be a hemodynamic signal which is acquired by the medical ventilation device through monitoring the ventilated object in the mechanical ventilation process. The hemodynamic signal has more direct correlation with the cardiogenic interference, which is beneficial to identifying the cardiogenic interference in the flow rate signal. For example, the hemodynamic signal includes at least one of an invasive blood pressure signal, an arterial blood pressure signal, a central venous pressure signal, and a pulse wave signal. **In** step S120, the fluctuation of the flow rate signal is identified according to the flow rate signal and the second signal. Since the cardiogenic interference can generate a fluctuation in both the flow rate signal and the second signal, the fluctuation characteristic in the flow rate signal can be amplified through the second signal, and then the fluctuation is identified. After identifying occurrence time of the fluctuation of the flow rate signal according to the amplified fluctuation characteristic, the cardiogenic interference in the fluctuation is identified subsequently based on the fluctuation characteristic of the fluctuation.

The fluctuation characteristic in the flow rate signal may be amplified based on the second signal in various signal processing manners, for example, a cross-correlation signal between the flow rate signal and the second signal may be acquired according to the flow rate signal and the second signal, and occurrence time of the fluctuation is determined according to the cross-correlation signal. Cross-correlation can enhance a response of the cardiogenic interference on the signal. For example, the cross-correlation signal between the flow rate signal and the second signal includes a second-order differential cross-correlation signal between the flow rate signal and the second signal, that is, a cross-correlation signal of a second-order differential signal of the flow rate signal and a second-order differential signal of the second signal. The second-order differential signal can interpret the fluctuation clearer and more intuitive.

Continuing taking the pressure signal as an example, the flow rate signal is F(t), the pressure signal is P(t), the second-order differential signals of the flow rate signal and the pressure signal are Δ(ΔF) and Δ(ΔP), respectively, and the second-order differential cross-correlation signal between the flow rate signal and the pressure signal is defined as: $Corr \left(n\right) = - {\sum }_{i = 1}^{N} Δ \left(ΔP\left(i\right)\right) \times Δ \left(ΔF\left(i\right)\right) ;$ where N is a length of the pressure signal and the flow rate signal, which length is used for calculating the cross-correlation signal. FIG. 2 illustrates an example of a flow rate signal, a pressure signal, and a second-order differential cross-correlation signal between the flow rate signal and the pressure signal. As shown in FIG. 2, the cross-correlation signal amplifies the fluctuation characteristic of the flow rate signal, and in the cross-correlation signal, one wave peak is correspondingly generated corresponding to one fluctuation. Through clinical data analysis, it is found that the wave peak of the cross-correlation signal generally appears at a certain time after a moment when the flow rate signal starts to increase obviously, which is generally about 200 ms after said moment. Therefore, according to the moment when the wave peak of the second-order differential cross-correlation signal between the pressure signal and the flow rate signal appears, the fluctuation moment of the flow rate signal can be identified. However, it can be understood that when other functions are used to calculate the cross-correlation signal, one wave valley may be correspondingly generated corresponding to one fluctuation, and then the fluctuation moment of the flow rate signal may be identified at the moment when the wave valley of the cross-correlation signal appears.

After the fluctuation in the flow rate signal is identified, at step S130, the fluctuation characteristic of the fluctuation are acquired, and the cardiogenic interference in the fluctuation is identified based on the fluctuation characteristic of the fluctuation. Due to the fact that the heartbeat cycle has certain regularity, the cardiogenic interference in the heartbeat cycle can be identified according to whether the fluctuation characteristic of the fluctuation has corresponding regularity. Furthermore, it can also be identified based on the fluctuation characteristic of the fluctuation that the fluctuation is caused by an invalid trigger, pipeline accumulated water or the cardiogenic interference.

The fluctuation characteristic of the fluctuation may include a time characteristic of the fluctuation, a shape characteristic of the fluctuation, and the likes. Exemplarily, the time characteristic of the fluctuation may include a duration of each fluctuation. For example, it may be determined that the fluctuation is the cardiogenic interference when the duration of the fluctuation is detected to be within a preset range. Since the duration of the fluctuation caused the cardiogenic interference is obviously greater than the duration of the fluctuation caused by the pipeline accumulated water, and is obviously smaller than the duration of the fluctuation caused by the invalid trigger. Accordingly, whether the fluctuation appears at the moment corresponding to the wave peak of the second-order differential cross-correlation signal is caused by the cardiogenic interference or not, can be determined according to the duration of the fluctuation, so that the fluctuation of the flow rate signal, which is caused by the pipeline accumulated water, the invalid trigger and the likes, is avoided from being mistakenly identified as that caused by the cardiogenic interference.

In one embodiment, the duration of each fluctuation may be determined according to a difference between an actual flow rate and an estimated flow rate. Specifically, the estimated flow rate of the ventilated object is determined firstly, and then the fluctuation signal of the flow rate is acquired according to a difference between the actual flow rate in the flow rate signal and the estimated flow rate at the same moment, and finally the duration of each fluctuation is obtained according to the fluctuation signal of the flow rate, For example, the duration of each fluctuation is obtained according to a time difference between two adjacent wave peaks or two adjacent wave valleys corresponding to each fluctuation in the fluctuation signal of the flow rate.

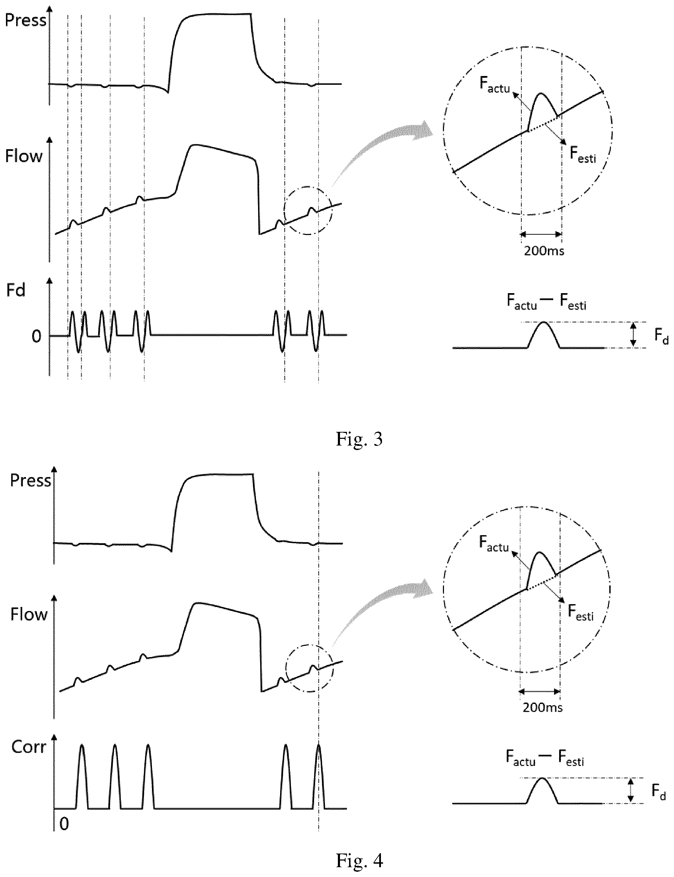

As shown in FIG. 3, the actual flow rate of the ventilated object is F_{actu}, the estimated flow rate of the ventilated object is Fₑₛₜᵢ, and a fluctuation amplitude of the flow rate is defined as F_{d}=F_{actu}-Fₑₛₜᵢ. The fluctuation amplitude of the flow rate describes a non-stability of the flow rate signal, and the greater the fluctuation amplitude, the more obvious an abrupt change of the flow rate signal is. Referring to waveforms of the flow rate signal and the fluctuation amplitude of the flow rate (F_{d}) of FIG. 3, it can be seen that corresponding to each fluctuation of the flow rate signal, one wave peak is generated a time period after a start moment and an end moment of the fluctuation, and the time difference between the corresponding moments of the two wave peaks is exactly equal to the time difference between the start moment and the end moment of the fluctuation of the flow rate signal. Therefore, the duration of each fluctuation can be obtained according to the time difference between two adjacent wave peaks corresponding to each fluctuation in the fluctuation signal of the flow rate, so as to determine whether the fluctuation is the cardiogenic interference.

It should be noted that in the example of FIG. 3, the fluctuation amplitude of the flow rate F_{d}=F_{actu}-Fₑₛₜᵢ, so that one wave peak is generated after the start moment and the end moment of each fluctuation in the fluctuation signal of the flow rate, and the duration of each fluctuation can be obtained according to the time difference between two adjacent wave peaks. In other embodiments, if the fluctuation amplitude of the flow rate F_{d} is defined as Fₑₛₜᵢ-F_{actu}, one wave valley is generated after the start moment and the end moment of each fluctuation in the fluctuation signal of the flow rate. At this time, the duration of each fluctuation may be obtained according to the time difference between two adjacent wave valleys.

To calculate the fluctuation amplitude of the flow rate F_{d}, it is necessary to determine the estimated flow rate Fₑₛₜᵢ for each time point. Exemplarily, a calculation method for estimating the flow rate Fₑₛₜᵢ includes determining the estimated flow rate at a current moment according to a respiratory mechanics model, based on an actual flow rate at a preset moment before the current moment. According to the respiratory mechanics model, since R (resistance) and C (compliance) are used to model the respiratory system of the human body, a typical first-order RC model is obtained, and according to characteristics of the first-order model, the theoretical estimated flow rate of an expiratory phase is Fₑₛₜᵢ(t)=PEF×e^{-t/τ}, wherein τ is an expiratory time constant, and PEF is an expiratory peak flow rate. According to a characteristic of exponential waveform, the estimated flow rate at any moment can be estimated based on the actual flow rate at a certain moment, that is, Fₑₛₜᵢ(t)=Flow(t-Δt)×e^{-Δt/τ}. For example, the current estimated flow rate may be estimated by using the above formula and the actual flow rate 100 100ms before the current moment, and the estimated flow rate obtained therefrom is more accurate than an estimated flow rate which is obtained by completely using the actual flow rate at the start moment for estimating the flow rate at each moment after the start moment.

Another calculation method for estimating the flow rate Fₑₛₜᵢ includes performing a linear fitting based on an actual flow rate within a preset time period before the current moment to determine an estimated flow rate at the current moment. This method performs a fitting based on historical flow rates within a time period according to a linear formula, and acquires the estimated flow rate at the current moment by using the linear formula, that is, Fₑₛₜᵢ (t)=k×t+b. For example, the estimated flow rate at the current moment may be linearly estimated by using actual flow rates within a 200 ms time period before the current moment.

In addition to the duration of each fluctuation, the time characteristic of the fluctuation may also include a rhythm of the fluctuation, i.e., a rhythm and law for occurrence time of multiple fluctuations. When multiple fluctuations conform to a preset rhythm, that is, the occurrence time of multiple fluctuations has a preset regularity, it is determined that the multiple fluctuations are cardiogenic interference. For example, the rhythm of the multiple fluctuations may include an average time interval of the multiple fluctuations, a variance of multiple time intervals of the multiple fluctuations, and the likes. When determining the rhythm of the multiple fluctuations, the rhythm of the multiple fluctuations can be determined according to the occurrence time of each fluctuation determined in step S120. Alternatively, since the occurrence time of the fluctuation is consistent with the rhythm of the wave peak of the cross-correlation signal, the rhythm of the multiple fluctuations can also be directly determined according to the cross-correlation signal.

In some embodiments, in order to improve the accuracy of cardiogenic interference identification, the cardiogenic interference in the fluctuation can also be jointly determined according to the duration of each fluctuation and the rhythm of multiple fluctuations. For example, when multiple fluctuations conform to a preset rhythm, and the duration of each fluctuation is within a preset range, the multiple fluctuations are determined as the cardiogenic interference.

In addition, the heartbeat cycle is generally stable, and if current cardiogenic interference is identified at one moment, when next cardiogenic interference occurs can be predicted according to the heartbeat cycle. The heartbeat cycle may be acquired according to the rhythm of the cardiogenic interference, or may be acquired in other manners. For example, the heartbeat cycle may be predicted according to a time interval between at least two occurrences of cardiogenic interference, and predicted time of the next cardiogenic interference may be acquired according to the occurrence time of the current cardiogenic interference and the heartbeat cycle.

In general, the characteristic of the cardiogenic interference in the flow rate signal and the pressure signal is more apparent in the exhalation phase as compared to the inhalation phase. Since the heartbeat is regular, the heartbeat characteristic in the exhalation phase may reflect the heartbeat characteristic of the entire ventilation period, and therefore, the cardiogenic interference may be identified in the exhalation phase, and the occurrence time of the cardiogenic interference in the inhalation phase can be predicted based on the identified result and the known heartbeat cycle.

Further, after identifying the cardiogenic interference in the flow rate signal, a magnitude of the cardiogenic interference can also be determined, and a trigger sensitivity can be adjusted according to the magnitude of the cardiogenic interference, thereby avoiding the false trigger caused by the cardiogenic interference. The trigger sensitivity can be adjusted according to the magnitude of the cardiogenic interference, so that the trigger sensitivity can be automatically adjusted along with the interference condition without manual adjustment by a user. Meanwhile, the trigger sensitivity can be adjusted according to the magnitude of the cardiogenic interference, so that the adjustment accuracy can be improved, the trigger sensitivity is prevented from being excessively improved, and the trigger sensitivity is ensured while keeping avoiding the false trigger caused by the cardiogenic interference. Exemplarily, the trigger sensitivity includes an inhalation trigger sensitivity or an exhalation trigger sensitivity, and the characterization forms thereof include, but are not limited to, a flow rate, a pressure, a change rate of the flow rate, a change rate of the pressure.

In order to adjust the trigger sensitivity according to the magnitude of the cardiogenic interference, it necessary to determine the magnitude of the cardiogenic interference firstly. In one embodiment, the fluctuation amplitude of the flow rate F_{d} at the occurrence time of the cardiogenic interference may be used as the magnitude of the cardiogenic interference. In other embodiments, the magnitude of the cardiogenic interference may also be determined according to other signal characteristics such as a waveform area of the fluctuation of the flow rate.

Specifically, the estimated flow rate of the ventilated object is determined, the fluctuation signal of the flow rate is acquired according to a difference between the actual flow rate in the flow rate signal and the estimated flow rate at the same moment, and then the magnitude of the cardiogenic interference is determined according to the fluctuation signal of the flow rate which corresponds to the occurrence time of the cardiogenic interference. For example, referring to FIG. 4, the occurrence time of the cardiogenic interference can be determined according to the cross-correlation signal, and a peak value of the fluctuation signal of the flow rate which corresponds to the occurrence time is determined as the magnitude of the cardiogenic interference. If the duration of each fluctuation is determined according to the fluctuation signal of the flow rate in step S120, the magnitude of the cardiogenic interference may be determined according to the fluctuation signal of the flow rate obtained in step S120. Otherwise, in step S130, only the fluctuation amplitude of the flow rate at the occurrence time of the cardiogenic interference may be calculated, so as to obtain the magnitude of the cardiogenic interference. The fluctuation amplitude of the flow rate F_{d} is the difference between the actual flow rate F_{actu} and the estimated flow rate Fₑₛₜᵢ. The actual flow rate F_{actu} may be directly extracted from the flow rate signal, and the calculation mode of the estimated flow rate Fₑₛₜᵢ may be referred to above, and details are not described herein.

Further, when calculating the magnitude of the cardiogenic interference to adjust the trigger sensitivity, a maximum value among a plurality of peak values in the fluctuation signal of the flow rate can be used as the magnitude of the cardiogenic interference, so as to avoid the false trigger caused by the cardiogenic interference as much as possible. Alternatively, an average value of the plurality of peak values in the fluctuation signal of the flow rate may also be used as the magnitude of the cardiogenic interference to reduce the error. The plurality of peak values may be in the same respiratory cycle, that is, the magnitude of the cardiogenic interference is calculated according to the plurality of cardiogenic interference of the same respiratory cycle, and the measurement of the magnitude of the primary interference is performed in each respiratory cycle, and the trigger sensitivity is adjusted in real time according to the measurement result. However, it should be noted that the plurality of peak values are not limited to the plurality of peak values in the same respiratory cycle, and they may be a plurality of peak values in at least two respiratory cycles, or a preset number of peak values. For example, the magnitude of the cardiogenic interference may be calculated every ten times of cardiogenic interference are detected.

After determining the magnitude of the cardiogenic interference, the trigger sensitivity may be adjusted to be greater than or equal to a trigger signal which is caused by the cardiogenic interference. For example, for the flow rate trigger, a trigger threshold S_{trig} may be adjusted to be greater than or equal to the magnitude F_{d} of the cardiogenic interference, thereby avoiding the false trigger caused by the cardiogenic interference. For example, the trigger threshold S_{trig} may be set to S_{trig}=F_{d}+k, where K is a constant or variable greater than zero.

In an embodiment, the trigger sensitivity at any moment during the ventilation process can be finally adjusted to be greater than or equal to the magnitude of the cardiogenic interference, so as to avoid the false trigger phenomenon as much as possible. When the adjusted object is an inhalation trigger threshold, the trigger sensitivity at any moment in the ventilation process may also be understood as an inhalation trigger threshold at any moment in an inhalation trigger phase.

Alternatively, since the heartbeat has regularity, when the occurrence time of the current cardiogenic interference is identified, the predicted time of the next cardiogenic interference can be obtained according to the occurrence time of the current cardiogenic interference and the heartbeat cycle, and the trigger sensitivity at a time which is close to the predicted time of the next cardiogenic interference is adjusted to be greater than or equal to the magnitude of the cardiogenic interference, which can also avoid false trigger caused by cardiogenic interference, and at the same time, be beneficial to ensure the sensitivity of the inhalation trigger. The time, which is close to the predicted time of the next cardiogenic interference, refers to a time period before and after the predicted time of the next cardiogenic interference, which predicted time is predicted according to the heartbeat cycle.

When the trigger sensitivity is adjusted according to the magnitude of the cardiogenic interference, the trigger sensitivity can be increased according to a preset step length until the trigger sensitivity is greater than or equal to the trigger signal which is caused by the cardiogenic interference, thereby avoiding an abrupt change of the trigger sensitivity. For example, the trigger sensitivity may be increased by a preset step length in each respiratory cycle, and each preset step length is a multiplied product of the magnitude of the cardiogenic interference F_{d} and a certain coefficient. Meanwhile, due to the fact that the magnitude of the cardiogenic interference can be continuously measured in the ventilation process, the preset step length can be adjusted in real time according to the magnitude of the cardiogenic interference.

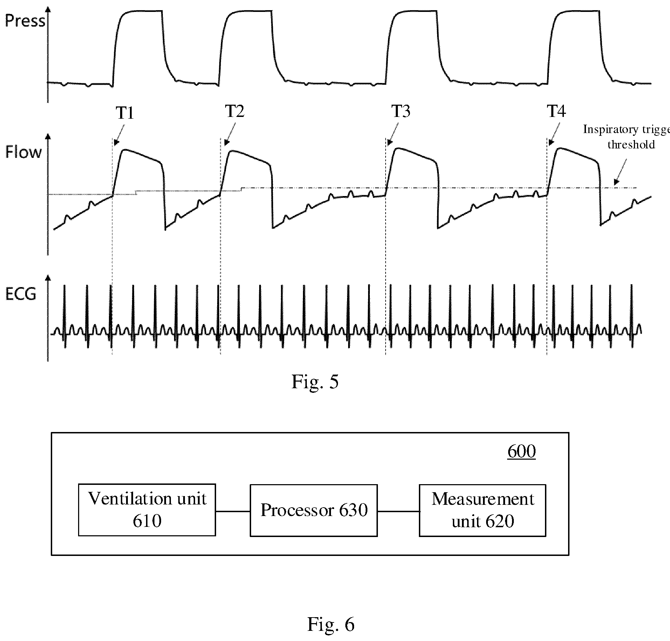

Referring to FIG. 5, a pressure signal, a flow rate signal, and an ECG signal, synchronously acquired, are shown. The ECG signal is used to illustrate the moment at which the cardiogenic interference of the flow rate signal occurs. At moment t1, a moment of the inhalation trigger is consistent with a moment of the cardiogenic interference, that is, the inhalation trigger at this moment is the false trigger caused by cardiogenic interference, so that the inhalation trigger threshold is increased according to the magnitude of the cardiogenic interference. In a next respiratory cycle, the moment T2 of the inhalation trigger is still consistent with the moment of the cardiogenic interference, and the inhalation trigger threshold is continuously increased until the inhalation trigger thresholds at moments T3 and T4 are greater than the magnitude of the cardiogenic interference, then the inhalation trigger is not related to the cardiogenic interference anymore.

Optionally, after the cardiogenic interference is identified, the cardiogenic interference in the flow rate signal can be indicated through a user interaction interface of the medical ventilation device, and be adjusted by a user manually based on the cardiogenic interference. When the inhalation trigger threshold is adjusted according to the magnitude of the cardiogenic interference, the adjustment may be guided according to a relationship between an inhalation trigger state of the medical ventilation device and the moment when the cardiogenic interference occurs. In the adjustment process, a correlation between the moment of the inhalation trigger and the moment of the cardiogenic interference can be determined, and if the correlation between the moment of the inhalation trigger and the moment of the cardiogenic interference is high, it is indicated that the inhalation trigger is caused by the cardiogenic interference. At this time, the trigger sensitivity is increased according to a certain step length until the correlation between the moment of the inhalation trigger and the moment of the cardiogenic interference is reduced to be insufficient to consider that inhalation trigger is the false trigger caused by the cardiogenic interference. Whether the adjustment is completed may be determined by the user according to the moment of the cardiogenic interference displayed on the interface. Alternatively, whether the adjustment is completed may be determined and indicated by the medical ventilation device according to whether the correlation between the moment of the inhalation trigger and the moment of the cardiogenic interference is reduced to a preset requirement.

To sum up, according to the identification method of cardiogenic interference 100 for a medical ventilation device according to the embodiments of this disclosure, the cardiogenic interference is identified based on physiological signals which are acquired by the medical ventilation device itself, without requiring physiological signals of external device, which has obvious clinical use values.

Another aspect of this disclosure provides a medical ventilation device, which is configured to replace, control, or change the respiration of a ventilated object, improve the respiratory function of the ventilated object by increasing the ventilation amount of the lungs, and reduce the respiratory consumption of the ventilated object. Referring to FIG. 6, the medical ventilation device 600 according to an embodiment of this disclosure includes a ventilation unit 610, a measurement unit 620, and a processor 630. The ventilation unit 610 is configured to provide ventilation to the ventilated object; the measurement unit 620 is configured to monitor the ventilated object during the ventilation to the ventilated object, so as to acquire a first signal and a second signal. The processor 630 is connected with the measurement unit 620 and is configured to acquire the first signal and the second signal. Only main functions of the medical ventilation device 600 are described below, and other specific details may be referred to above. The medical ventilation device 600 may be implemented as a medical device having a mechanical ventilation function, such as a ventilator or an anesthesia machine.

Exemplarily, the ventilation unit 610 includes a gas source and a gas path, and the gas path includes an inspiratory branch and an expiratory path. The gas source is configured to provide a gas during the mechanical ventilation, and the inspiratory branch is connected with the gas source to provide an inspiratory path during the mechanical ventilation; and the expiratory path is configured to provide an exhalation path during the mechanical ventilation. The gas supplied to the ventilated object by the ventilation unit 610 may be oxygen, or a mixed gas of air and oxygen. The gas may be a compressed gas provided a gas supply system, or may be a gas derived from the environment.

The measurement unit 620 includes a sensor for measuring the first signal and the second signal during the mechanical ventilation. The first signal includes a flow rate signal, and the sensor includes at least a flow rate sensor for measuring the flow rate signal. The second signal may include a pressure signal which reflects an inspiratory effort of the ventilated object, and the pressure signal includes at least one of an intrapulmonary pressure signal, an airway pressure signal, an esophageal pressure signal, a transdiaphragmatic pressure signal, an intragastric pressure signal, and a carina pressure signal. The sensor also includes a pressure sensor for measuring the pressure signal. Exemplarily, the flow rate sensor is disposed at an exhalation end and an inhalation end of the gas path, and the pressure sensor is disposed at an end connected with the ventilated object. The second signal may further include a hemodynamic signal, which includes at least one of an invasive blood pressure signal, an arterial blood pressure signal, a central venous pressure signal, and a pulse wave signal. The measurement unit 620 includes a measurement apparatus for measuring the hemodynamic signal. The second signal may further include a respiratory system signal which reflects the inspiratory effort of the ventilated object, and specifically includes at least one of a lung ultrasound imaging signal, a diaphragm ultrasound imaging signal, an impedance imaging signal, and a diaphragm electrical signal. The measurement unit 620 includes a measurement apparatus for measuring a respiratory system signal which reflects the inspiratory effort of the ventilated object.

The processor 630 is connected to the measurement unit 620, and is configured to receive the first signal and the second signal which are measured by the measurement unit 620, wherein the first signal includes a flow rate signal; to identify a fluctuation of the flow rate signal according to the flow rate signal and the second signal; to acquire a fluctuation characteristic of the fluctuation, and to identify the cardiogenic interference in the fluctuation based on the fluctuation characteristic of the fluctuation.

The processor 630 may be implemented by software, hardware, firmware, or any combination thereof, may use circuits, a single or multiple application-specific integrated circuits, a single or multiple general-purpose integrated circuits, a single or multiple microprocessors, a single or multiple programmable logic devices, or any combination of the foregoing circuits and/or devices, or other suitable circuits or devices, and the processor 630 may control other components in the medical ventilation device 600 to perform desired functions.

In an embodiment, the processor 630 is further configured to determine a magnitude of the cardiogenic interference; and to adjust a trigger sensitivity according to the magnitude of the cardiogenic interference.

In an embodiment, identifying a fluctuation of the flow rate signal according to the flow rate signal and the second signal includes: acquiring a cross-correlation signal between the flow rate signal and the second signal according to the flow rate signal and the second signal; and determining occurrence time of the fluctuation according to the cross-correlation signal. Exemplarily, the cross-correlation signal includes a second-order differential cross-correlation signal.

In one embodiment, the fluctuation characteristic of the fluctuation includes a time characteristic of the fluctuation. Further, the time characteristic of the fluctuation includes at least one of: a duration of each fluctuation, a rhythm of the fluctuation. Identifying the cardiogenic interference in the fluctuation based on the duration of each fluctuation, includes determining that the fluctuation is the cardiogenic interference when the duration of the fluctuation is within a preset range. Identifying the cardiogenic interference in the fluctuation based on the rhythm of the fluctuation, includes determining that multiple fluctuations are the cardiogenic interference, when the multiple fluctuations conform to a preset rhythm.

In one embodiment, the duration of each fluctuation includes determining an estimated flow rate of the ventilated object is acquired by acquiring a fluctuation signal of a flow rate according to a difference between an actual flow rate in the flow rate signal and the estimated flow rate at a same moment; and obtaining the duration of each fluctuation according to the fluctuation signal of the flow rate.

In one embodiment, determining a magnitude of the cardiogenic interference includes determining an estimated flow rate of the ventilated object; acquiring a fluctuation signal of a flow rate according to a difference between an actual flow rate in the flow rate signal and the estimated flow rate at a same moment; and determining the magnitude of the cardiogenic interference according to the fluctuation signal of the flow rate which corresponds to occurrence time of the cardiogenic interference.

Exemplarily, determining the estimated flow rate of the ventilated object includes determining the estimated flow rate at a current moment according to a respiratory mechanics model, based on an actual flow rate at a preset moment before the current moment. Alternatively, determining the estimated flow rate of the ventilated object includes performing a linear fitting based on an actual flow rate within a preset time period before a current moment, so as to determine the estimated flow rate at the current moment.

In one embodiment, adjusting an inhalation trigger threshold according to the magnitude of the cardiogenic interference includes increasing the trigger sensitivity according to a preset step length until the trigger sensitivity is greater than or equal to a trigger signal which is caused by the cardiogenic interference. Further, the preset step length may be adjusted in real time according to the magnitude of the cardiogenic interference.

In one embodiment, adjusting the trigger sensitivity according to the magnitude of the cardiogenic interference includes: adjusting the trigger sensitivity at any moment in the ventilation process to be greater than or equal to the magnitude of the cardiogenic interference; or determining predicted time of next cardiogenic interference according to occurrence time of current cardiogenic interference, and adjusting the trigger sensitivity at a time which is close to the predicted time of the next cardiogenic interference to be greater than or equal to the magnitude of the cardiogenic interference.

According to an embodiment of this disclosure, the medical ventilation device 600 identifies the cardiogenic interference in the flow rate signal based on the signal acquired by the medical ventilation device 600 itself, without requiring a signal of an external device, which has an obvious clinical use value.

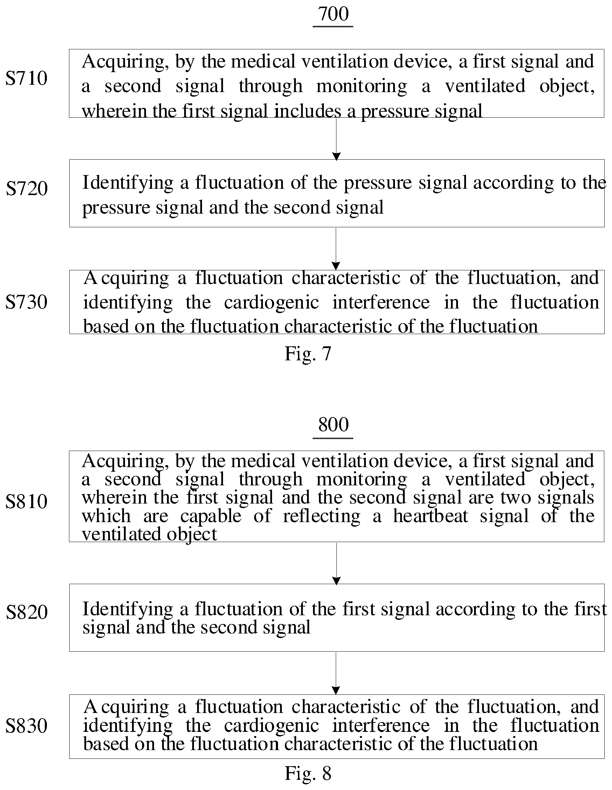

Hereinafter, an identification method of cardiogenic interference for a medical ventilation device according to another embodiment of this disclosure is described with reference to FIG. 7. FIG. 7 is a schematic flowchart of an identification method of cardiogenic interference 700 according to an embodiment of this disclosure.

As shown in FIG. 7, the identification method of cardiogenic interference 700 in this embodiment of this disclosure includes the following steps.

In step S710, a first signal and a second signal are acquired by a medical ventilation device through monitoring a ventilated object, wherein the first signal includes a pressure signal.

In step S720, a fluctuation of the pressure signal is identified according to the pressure signal and the second signal.

In step S730, a fluctuation characteristic of the fluctuation is acquired, and the cardiogenic interference in the fluctuation is identified based on the fluctuation characteristic of the fluctuation.

As mentioned above, the modes for the inhalation trigger of the medical ventilation device includes a flow rate trigger mode and a pressure trigger mode, and the identification method of cardiogenic interference 100 of the previous embodiment can identify the cardiogenic interference in the flow rate signal and is mainly suitable for flow rate trigger. The identification method of cardiogenic interference 700 of the present embodiment can identify the cardiogenic interference in the pressure signal, and is mainly suitable for the pressure trigger. The following describes only the main steps of the identification method of cardiogenic interference 700, and more details may be referred to above.

Due to the systolic and diastolic process of the heart, a change in the flow rate is caused, so that the second signal acquired in step S710 may include a flow rate signal. Thereafter, in step S720, the fluctuation caused by the cardiogenic interference in the pressure signal may be amplified by the second signal, thereby identifying the fluctuation in the pressure signal. For example, a cross-correlation signal between the flow rate signal and the pressure signal may be obtained according to the second signal and the pressure signal; and occurrence time of the fluctuation is determined according to the cross-correlation signal. Exemplarily, the cross-correlation signal includes a second-order differential cross-correlation signal between the flow rate signal and the pressure signal.

After the fluctuation of the pressure signal is identified, in step S730, the fluctuation characteristic of the fluctuation are acquired, and the cardiogenic interference is identified based on the fluctuation characteristic of the fluctuation. The fluctuation characteristic may include a time characteristic of the fluctuation. The time characteristic of the fluctuation includes at least one of: a duration of each fluctuation, a rhythm of the fluctuation. For example, when the duration of the fluctuation is within a preset range, it is determined that the fluctuation is the cardiogenic interference. Alternatively, when multiple fluctuations conform to a preset rhythm, it is determined that the multiple fluctuations are multiple cardiogenic interference.

The rhythm of multiple fluctuations may be determined directly according to wave peaks or wave valleys of the cross-correlation signal, and the duration of each fluctuation may be determined by determining an estimated pressure of the ventilated object, obtaining a fluctuation signal of a pressure according to a difference between an actual pressure in the pressure signal and the estimated pressure at a same moment; and obtaining the duration of each fluctuation according to the fluctuation signal of the pressure. For example, the duration of each fluctuation can be obtained according to a time difference between two adjacent wave peaks or two adjacent wave valleys which corresponds to each fluctuation in the fluctuation signal of the pressure. One way of determining the estimated pressure of the ventilated object is determining the estimated pressure at a current moment according to a respiratory mechanics model based on an actual pressure at a preset moment before the current moment. Another way of determining the estimated pressure of the ventilated object is performing a linear fitting based on an actual pressure within a preset time period before a current moment, so as to determine the estimated pressure at the current moment.

After the cardiogenic interference is identified, a magnitude of the cardiogenic interference can be further determined, and a trigger sensitivity is adjusted according to the magnitude of the cardiogenic interference, wherein the trigger sensitivity here is the trigger sensitivity of the pressure trigger. The magnitude of the cardiogenic interference may be determined according to the fluctuation signal of the pressure described above. Exemplarily, the magnitude of the cardiogenic interference includes a maximum or average value of multiple peak values in the fluctuation signal of the pressure.

In one embodiment, when the trigger sensitivity is adjusted according to the magnitude of the cardiogenic interference, the trigger sensitivity may be increased according to a preset step length until the trigger sensitivity is greater than or equal to a trigger signal which is caused by the cardiogenic interference. The preset step length may be adjusted in real time according to the magnitude of the cardiogenic interference.

When the trigger sensitivity is adjusted according to the magnitude of the cardiogenic interference, the trigger sensitivity at any moment in the ventilation process can be adjusted to be greater than or equal to the magnitude of the cardiogenic interference; or predicted time of next cardiogenic interference is determined according to occurrence time of current cardiogenic interference, and the trigger sensitivity at a time, which is close to the predicted time of next cardiogenic interference, is adjusted to be greater than or equal to the magnitude of the cardiogenic interference. When determining the predicted time of the next cardiogenic interference according to the occurrence time of the current cardiogenic interference, a heartbeat cycle may be predicted according to a time interval between at least two times of cardiogenic interference, and the predicted time of the next cardiogenic interference may be obtained according to the occurrence time of the current cardiogenic interference and the heartbeat cycle.

Another aspect of this disclosure provides a medical ventilation device, wherein the medical ventilation device may implement the above-mentioned identification method of cardiogenic interference 700. Continue to refer to FIG. 6, the medical ventilation device 600 in this embodiment of this disclosure includes a ventilation unit 610, a measurement unit 620, and a processor 630. The ventilation unit 610 is configured to provide ventilation to the ventilated object; the measurement unit 620 is configured to monitor the ventilated object during the ventilation to the ventilated object, so as to acquire a first signal and a second signal, wherein the first signal includes a pressure signal, and the second signal includes, but is not limited to, a flow rate signal. The processor 630 is connected with the measurement unit 620 and is configured to acquire the first signal and the second signal., and the processor 630 is further configured to execute the above-mentioned identification method of cardiogenic interference 700, that is, acquiring a first signal and a second signal, wherein the first signal includes a pressure signal; identifying a fluctuation of the pressure signal according to the pressure signal and the second signal; and acquiring a fluctuation characteristic of the fluctuation, and identifying the cardiogenic interference in the fluctuation based on the fluctuation characteristic of the fluctuation.

The identification method of cardiogenic interference 700 and the medical ventilation device of the present embodiment identify the cardiogenic interference in the pressure signal based on the signal which are acquired during monitoring the ventilated object by the medical ventilation device itself, and can also identify the cardiogenic interference when there is no external device for monitoring the heartbeat information, thereby avoiding the false trigger caused by the cardiogenic interference.

Hereinafter, an identification method of cardiogenic interference for a medical ventilation device according to another embodiment of this disclosure is described with reference to FIG. 8. FIG. 8 is a schematic flowchart of an identification method of cardiogenic interference 800 according to an embodiment of this disclosure.

As shown in FIG. 8, the identification method of cardiogenic interference 800 in this embodiment of this disclosure includes the following steps.

In step S810, a first signal and a second signal are acquired by a medical ventilation device through monitoring a ventilated object, wherein the first signal and the second signal are two signals which are capable of reflecting a heartbeat signal of the ventilated object.

In step S820, a fluctuation of the first signal is identified according to the first signal and the second signal.

In step S830, a fluctuation characteristic of the fluctuation is acquired, and the cardiogenic interference in the fluctuation is identified based on the fluctuation characteristic of the fluctuation.

Exemplarily, in step S810, the first signal and the second signal are arbitrary two signals acquired by the medical ventilation device through monitoring the ventilated object, including but not limited to a flow rate signal, a pressure signal, a hemodynamic signal, a respiratory system signal which reflects an inspiratory effort of the ventilated object, and the likes. Wherein the pressure signal includes, but is not limited to, an intrapulmonary pressure signal, an airway pressure signal, an esophageal pressure signal, a transdiaphragmatic pressure signal, an intragastric pressure signal, and a carina pressure signal. The hemodynamic signal includes, but is not limited to, an invasive blood pressure signal, an arterial blood pressure signal, a central venous pressure signal, and a pulse wave signal. The respiratory system signal, which reflects an inspiratory effort of the ventilated object, includes, but is not limited to, a lung ultrasound imaging signal, a diaphragm ultrasound imaging signal, an impedance imaging signal, and a diaphragm electrical signal.

Exemplarily, identifying the fluctuation of the first signal according to the first signal and the second signal includes: acquiring a cross-correlation signal between the first signal and the second signal according to the first signal and the second signal, and determining occurrence time of the fluctuation according to the cross-correlation signal. After the fluctuation of the first signal is identified, the cardiogenic interference in the fluctuation is identified according to a fluctuation characteristic of the fluctuation. For example, the fluctuation characteristic of the fluctuation includes a time characteristic of the fluctuation, and the time characteristic of the fluctuation includes at least one of a duration of each fluctuation and a rhythm of the fluctuation. For example, when the duration of a fluctuation is within a preset range, it is determined that the fluctuation is the cardiogenic interference. Alternatively, when multiple fluctuations conform to a preset rhythm, it is determined that the multiple fluctuations are the cardiogenic interference.

Exemplarily, after identifying the cardiogenic interference, the method further includes determining a magnitude of the cardiogenic interference; and adjusting a trigger sensitivity of the medical ventilation device according to the magnitude of the cardiogenic interference. Exemplarily, the trigger sensitivity of the medical ventilation device is a trigger sensitivity related to the first signal.

Another aspect of this disclosure provides a medical ventilation device, wherein the medical ventilation device may implement the above-mentioned identification method of cardiogenic interference 800. Continue to refer to FIG. 6, the medical ventilation device 600 in this embodiment of this disclosure includes a ventilation unit 610, a measurement unit 620, and a processor 630, and the ventilation unit 610 is configured to provide ventilation to the ventilated object; the measurement unit 620 is configured to monitor the ventilated object during the ventilation to the ventilated object, so as to acquire a first signal and a second signal. The first signal and the second signal are two signals which are capable of reflecting a heartbeat signal of the ventilated object. The processor 630 is connected with the measurement unit 620 and is configured to acquire the first signal and the second signal. The processor 630 is further configured to implement the above-mentioned identification method of cardiogenic interference 800, that is, acquiring a first signal and a second signal, identifying a fluctuation of the first signal, and further identifying the cardiogenic interference in the fluctuation based on the second signal.

The identification method of cardiogenic interference 800 and the medical ventilation device of the present embodiment identify the cardiogenic interference in one of two signals based on the two signals that can reflect the heartbeat signal of the ventilated object and are obtained through monitoring the ventilated object by the medical ventilation device itself, and further identify the cardiogenic interference when there is no external device for monitoring the heartbeat information, thereby avoiding the false trigger caused by the cardiogenic interference.

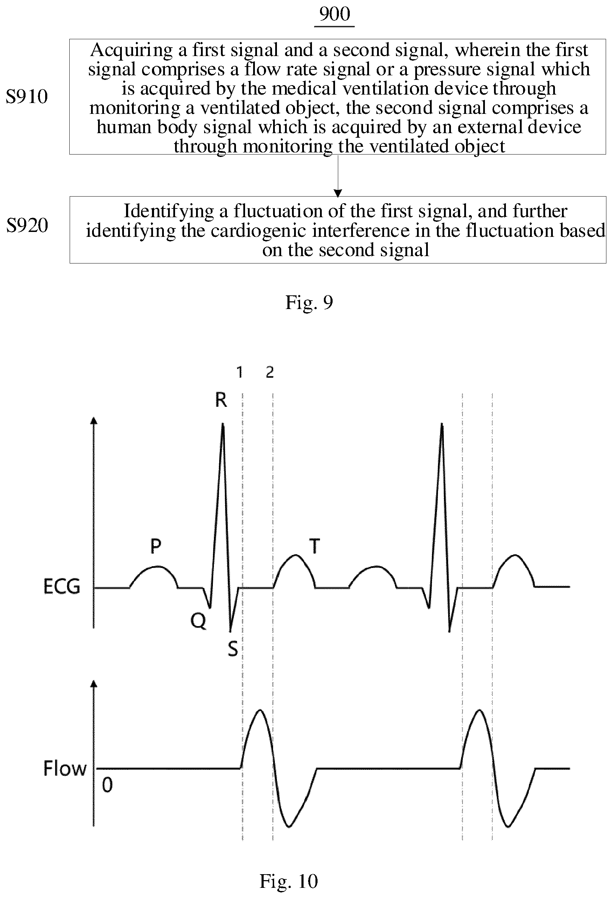

Another aspect of this disclosure provides an identification method of cardiogenic interference for a medical ventilation device, referring to FIG. 9, the identification method of cardiogenic interference 900 includes the following steps.

In step S910, a first signal and a second signal are acquired, wherein the first signal includes a flow rate signal or a pressure signal, which are acquired by a medical ventilation device through monitoring a ventilated object, and the second signal includes a human body signal which is acquired by an external device through monitoring the ventilated object.

At step S920, a fluctuation of the flow rate signal is identified, and a cardiogenic interference in the fluctuation is identified based on the second signal. The identification method of cardiogenic interference 900 according to the present embodiment obtains the second signal from the external device, and identifies the cardiogenic interference in the flow rate signal or the pressure signal based on the second signal.

In one embodiment, after identifying the cardiogenic interference, the method further includes: determining a magnitude of the cardiogenic interference, and adjusting a trigger sensitivity according to the magnitude of the cardiogenic interference, without requiring a user to manually adjust the trigger sensitivity, and ensuring the inhalation trigger sensitivity while the avoiding false trigger caused by the cardiogenic interference.

In one embodiment, identifying the cardiogenic interference based on the second signal includes: acquiring a predicted time range in which the cardiogenic interference occurs based on the second signal; and determining the fluctuation within the predicted time range as the cardiogenic interference. The second signal may be a physiological signal associated with a heartbeat. Further, the second signal may be at least one of a signal capable of directly reflecting a heartbeat characteristic of the ventilated object, for example, an ECG signal (ECG), a pulse wave signal, and an invasive blood pressure signal. Since the second signal can directly reflect the heartbeat characteristic, the occurrence time and duration of the heartbeat can be identified according to the characteristic reflecting the heartbeat in the second signal, so as to acquire the predicted time range of the cardiogenic interference.

Taking an electrocardiographic signal as an example, referring to FIG. 10, a series of waveforms may be recorded on the electrocardiogram in one cardiac cycle, including P wave, QRS wave group, and T wave. It is generally considered that within 30-60 ms after an end of one QRS wave group, that is, between time periods 1 and 2 in FIG. 10, the flow rate signal is significantly increased and then decreased, that is, the cardiogenic interference, and the increased flow rate of this process is easily to cause the false trigger. Therefore, according to the end moment of the QRS wave group in the electrocardiographic signal, the predicted time range of the cardiogenic interference can be obtained, and the fluctuation of the flow rate in the predicted time range is determined as the cardiogenic interference.

In another embodiment, the second signal may be a respiratory system signal, which reflects the inspiratory effort of the ventilated object and is acquired by the external device, and the second signal may include at least one of a lung ultrasound imaging signal, a diaphragm ultrasound imaging signal, an impedance imaging signal, and a diaphragm electrical signal.

After identifying the magnitude of the cardiogenic interference, the magnitude of the cardiogenic interference may also be determined, and the inspiratory trigger threshold may be adjusted according to the magnitude of the cardiogenic interference. Exemplarily, determining the magnitude of the cardiogenic interference includes: determining an estimated flow rate of the ventilated object; acquiring a fluctuation signal of a flow rate according to a difference between an actual flow rate in the flow rate signal and the estimated flow rate at the same moment; and determining the magnitude of the cardiogenic interference according to the fluctuation signal of the flow rate which corresponds to occurrence time of the cardiogenic interference. Determining the estimated flow rate of the ventilated object includes determining the estimated flow rate at a current moment according to a respiratory mechanics model based on an actual flow rate at a preset moment before the current moment; or performing a linear fitting based on an actual flow rate in a preset time period before a current moment, so as to determine the estimated flow rate at the current moment. Adjusting the trigger sensitivity according to the magnitude of the cardiogenic interference includes increasing the trigger sensitivity according to a preset step length until the trigger sensitivity is greater than or equal to the trigger signal which is caused by the cardiogenic interference. The preset step length may be adjusted in real time according to the magnitude of the cardiogenic interference. When the trigger sensitivity is adjusted according to the magnitude of the cardiogenic interference, the trigger sensitivity at any moment in the ventilation process can be adjusted to be greater than or equal to the magnitude of the cardiogenic interference, or the trigger sensitivity at a time which is close to the predicted moment of the cardiogenic interference is adjusted to be greater than or equal to the magnitude of the cardiogenic interference. The specific details of step S930 and step S940 may refer to the related description in the identification method of cardiogenic interference 100, and details are not described herein.

Another aspect of this disclosure provides a medical ventilation device, wherein the medical ventilation device may implement the above-mentioned identification method of cardiogenic interference 900. Continue to refer to FIG. 6, the medical ventilation device 600 in this embodiment of this disclosure includes a ventilation unit 610, a measurement unit 620, and a processor 630, wherein the ventilation unit 610 is configured to provide ventilation for the ventilated object. the measurement unit 620 is configured to monitor the ventilated object during the ventilation, so as to acquire a first signal. The processor 630 is connected with the measurement unit 620 and the external device, and is configured to acquire the first signal, and to acquire a second signal obtained by the external device through monitoring the ventilated object, and the processor 630 is further configured to execute the identification method of cardiogenic interference 900.

In this embodiment, the identification method of cardiogenic interference 900 and the medical ventilation device identify the cardiogenic interference in the flow rate signal based on the signal which is acquired by the external device through monitoring the ventilated object, thereby improving the identification accuracy of the cardiogenic interference.

Although the exemplary embodiments are described here with reference to the accompanying drawings, it should be understood that the aforementioned exemplary embodiments are only illustrative and are not intended to limit the scope of this disclosure. Ordinary technical personnel in this field can make various changes and modifications without deviating from the scope and spirit of this disclosure. All these changes and modifications are intended to be included within the scope of this disclosure as claimed in the attached claims.

Ordinary technical personnel in this field can realize that the units and algorithm steps described in combination with this disclosure can be implemented in electronic hardware, or a combination of computer software and electronic hardware. Whether these functions are executed in hardware or software depends on the specific application and design constraints of the technical solution. Professional technicians may use different methods to implement the described functions for each specific application, but such implementation should not be considered beyond the scope of this disclosure.

In the several embodiments provided in this disclosure, it should be understood that the disclosed devices and methods can be implemented in other ways. For example, the device embodiments described above are only schematic. For example, the division of the units is only a logical functional division, and there may be other division methods in actual implementations. For example, multiple units or components can be combined or integrated into another device, or some characteristics can be ignored or not executed.

The disclosure provided here provides a large number of specific details. However, it can be understood that the embodiments of this disclosure can be practiced without these specific details. In some examples, well-known methods, structures, and techniques are not shown in detail to avoid blurring the understanding of this disclosure.

Similarly, it should be understood that in order to streamline this disclosure and assist in understanding one or more of the various aspects of this disclosure, in the description of exemplary embodiments of this disclosure, the various characteristics of this disclosure are sometimes grouped together into a single embodiment, diagram, or description thereof. However, the method of this disclosure should not be interpreted as reflecting the intention that the claimed protection of this disclosure requires more characteristics than those explicitly recorded in each claim. More precisely, as reflected in the corresponding claims, the inventive point is that the corresponding technical problem can be solved with less than all characteristics of a single disclosed embodiment. Therefore, the claims following the specific implementation method are explicitly incorporated into the specific implementation method, where each claim itself serves as a separate embodiment of this disclosure.

Ordinary technical personnel in this field can understand that, in addition to mutual exclusion between characteristics, any combination can be configured to combine all characteristics disclosed in this disclosure (including accompanying claims, abstract, and drawings), as well as all processes or units of any method or device so disclosed. Unless otherwise explicitly stated, each characteristic disclosed in this disclosure (including accompanying claims, abstract, and accompanying drawings) may be replaced by alternative characteristics that provide the same, equivalent, or similar purpose.

In addition, Ordinary technical personnel in this field can understand that although some embodiments described herein include certain characteristics rather than other characteristics included in other embodiments, the combination of characteristics of different embodiments means that they are within the scope of this disclosure and form different embodiments.

The various component embodiments of this disclosure can be implemented in hardware, software modules running on one or more processors, or a combination thereof. Ordinary technical personnel in this field should understand that a microprocessor or digital signal processor (DSP) can be used in practice to implement some or all of the functions of some modules according to the embodiments of this disclosure. This disclosure can also be implemented as a partial or complete device program (such as a computer program and a computer program product) for executing the method described herein. The program implementing this disclosure can be stored at a computer-readable medium or can have the form of one or more signals. Such signals can be downloaded from internet websites, provided on carrier signals, or in any other form.

It should be noted that the above embodiments illustrate this disclosure rather than limit it, and Ordinary technical personnel in this field can design alternative embodiments without departing from the scope of the technology

The above is only the specific implementation method or explanation of the specific implementation method of this disclosure. The protection scope of this disclosure is not limited to this. Any technical personnel familiar with the technical field can easily think of changes or replacements within the technical scope disclosed in this disclosure, and those changes or replacements should fall into the protection scope of this disclosure. The protection scope of this disclosure shall be based on the protection scope of the claims.

## Claims

1. An identification software for implementing an identification method of cardiogenic interference for a medical ventilation device, **characterized in that**, said software comprising code for processing by said medical ventilation device for:
acquiring a first signal by the medical ventilation device through monitoring a ventilated object, wherein the first signal comprises a flow rate signal or a pressure signal;
acquiring a second signal by the medical ventilation device through monitoring the ventilated object;
identifying a fluctuation of the first signal according to the first signal and the second signal; and
acquiring a fluctuation characteristic of the fluctuation, and identifying the cardiogenic interference in the fluctuation based on the fluctuation characteristic of the fluctuation.

2. The identification software of cardiogenic interference according to claim 1, **characterized in that**, the fluctuation characteristic of the fluctuation comprises a time feature of the fluctuation;
preferably, the time feature of the fluctuation comprises at least one of a duration of each fluctuation and a rhythm of the fluctuation.

3. The identification software of cardiogenic interference according to claim 1, **characterized in that**, further comprising:
determining a magnitude of the cardiogenic interference; and
adjusting a trigger sensitivity according to the magnitude of the cardiogenic interference.

4. The identification software of cardiogenic interference according to claim 1, **characterized in that**, when the first signal comprises the flow rate signal, the second signal comprises a pressure signal;
preferably, the pressure signal reflects an inspiratory effort of the ventilated object;
more preferably, the pressure signal, which reflects an inspiratory effort of the ventilated object, comprises at least one of an intrapulmonary pressure signal, an airway pressure signal, an esophageal pressure signal, a transdiaphragmatic pressure signal, an intragastric pressure signal, and a carina pressure signal.

5. The identification software of cardiogenic interference according to claim 1, **characterized in that**, when the first signal comprises the flow rate signal, the second signal comprises a hemodynamic signal;
more preferably, the hemodynamic signal comprises at least one of an invasive blood pressure signal, an arterial blood pressure signal, a central venous pressure signal, and a pulse wave signal.

6. The identification software of cardiogenic interference according to claim 1, **characterized in that**, when the first signal comprises the flow rate signal, the second signal comprises a respiratory system signal, which reflects an inspiratory effort of the ventilated object;
preferably, the respiratory system signal, which reflects the inspiratory effort of the ventilated object, comprises at least one of a lung ultrasound imaging signal, a diaphragm ultrasound imaging signal, an impedance imaging signal, and a diaphragm electrical signal.

7. The identification software of cardiogenic interference according to claim 1, **characterized in that**, when the first signal comprises the pressure signal, the second signal comprises a flow rate signal.

8. The identification software of cardiogenic interference according to any one of claims 1-7, **characterized in that**, identifying a fluctuation of the first signal according to the first signal and the second signal comprises:
acquiring a cross-correlation signal between the first signal and the second signal according to the first signal and the second signal; and
determining an occurrence time of the fluctuation according to the cross-correlation signal.

9. The identification software of cardiogenic interference according to claim 2, **characterized in that**, identifying the cardiogenic interference in the fluctuation based on the fluctuation characteristic of the fluctuation comprises:
determining that a fluctuation is the cardiogenic interference when a duration of said fluctuation is within a preset range; or
identifying the cardiogenic interference in the fluctuation based on the time feature of the fluctuation comprises:
determining that multiple fluctuations are the cardiogenic interference, when said multiple fluctuations conform to a preset rhythm.

10. The identification software of cardiogenic interference according to claim 2, **characterized in that**, when the first signal comprises the flow rate signal, acquiring the duration of each fluctuation comprises:
determining an estimated flow rate of the ventilated object;
acquiring a fluctuation signal of a flow rate according to a difference between an actual flow rate in the flow rate signal and a corresponding estimated flow rate; and
acquiring the duration of each fluctuation according to the fluctuation signal of the flow rate.

11. The identification software of cardiogenic interference according to claim 3, **characterized in that**, when the first signal comprises the flow rate signal, determining a magnitude of the cardiogenic interference comprises:
determining an estimated flow rate of the ventilated object;
acquiring a fluctuation signal of a flow rate according to a difference between an actual flow rate in the flow rate signal and a corresponding estimated flow rate; and
determining an occurrence time of the cardiogenic interference, and further determining the magnitude of the cardiogenic interference according to the fluctuation signal of the flow rate, which fluctuation signal corresponds to the occurrence time of the cardiogenic interference.

12. The identification software of cardiogenic interference according to claim 10 or 11, **characterized in that**, determining an estimated flow rate of the ventilated object comprises:
determining the estimated flow rate at a current moment according to a respiratory mechanics model, based on an actual flow rate at a preset moment before the current moment; or
performing a linear fitting based on an actual flow rate within a preset time period before a current moment, so as to determine the estimated flow rate at the current moment.

13. The identification software of cardiogenic interference according to claim 3, **characterized in that**, adjusting a trigger sensitivity according to the magnitude of the cardiogenic interference comprises:
increasing the trigger sensitivity according to a preset step length until the trigger sensitivity is greater than or equal to a trigger signal which is caused by the cardiogenic interference;
preferably, the method further comprises adjusting the preset step length in real time according to the magnitude of the cardiogenic interference.

14. The identification software of cardiogenic interference according to claim 3, **characterized in that**, adjusting a trigger sensitivity according to the magnitude of the cardiogenic interference comprises:
adjusting respectively trigger sensitivities during a ventilation process to be greater than or equal to the magnitude of the cardiogenic interference; or
determining a predicted time of next cardiogenic interference according to an occurrence time of current cardiogenic interference, and adjusting the trigger sensitivity at a time, which time is close to the predicted time of the next cardiogenic interference, to be greater than or equal to the magnitude of the current cardiogenic interference;
preferably, the method further comprises predicting a heartbeat cycle according to a time interval between at least two occurrences of cardiogenic interference; and
acquiring the predicted time of the next cardiogenic interference according to the occurrence time of the current cardiogenic interference and the heartbeat cycle.

15. A medical ventilation device, (600) including:
a ventilation unit (610), which is configured to provide ventilation to a ventilated object;
a measurement unit (620), which is configured to monitor the ventilated object during the ventilation to the ventilated object, so as to acquire a first signal and a second signal; and
a processor (630), which is connected with the measurement unit (620) and configured to acquire the first signal and the second signal, wherein the processor (630) is further configured to execute the identification software of any of claims 1 to 14 for identifying the cardiogenic interference.

## Patentansprüche

1. Eine Identifizierungssoftware zur Umsetzung eines Verfahrens zur Erkennung kardiogener Interferenzen für eine medizinisches Beatmungsvorrichtung, **dadurch gekennzeichnet, dass** die Software einen Code zur Verarbeitung durch die medizinische Beatmungsvorrichtung umfasst, und zwar für:
das Erfassen eines ersten Signals durch die medizinische Beatmungsvorrichtung mittels Überwachung eines beatmeten Patienten, wobei das erste Signal ein Durchflusssignal oder ein Drucksignal umfasst;
das Erfassen eines zweiten Signals durch die medizinische Beatmungsvorrichtung mittels Überwachung des beatmeten Patienten;
das Erkennen einer Schwankung des ersten Signals anhand des ersten Signals und des zweiten Signals; und
das Erfassen einer Charakteristik der Schwankung und das Identifizieren der kardiogenen Interferenz in der Schwankung auf der Grundlage der Charakteristik der Schwankung.

2. Die Software zur Erkennung kardiogener Interferenzen nach Anspruch 1, **dadurch gekennzeichnet, dass** die Charakteristik der Schwankung ein Zeitmerkmal der Schwankung umfasst;
vorzugsweise umfasst das zeitliche Merkmal der Schwankung mindestens eines der folgenden Elemente: die Dauer jeder Schwankung oder den Rhythmus der Schwankung.

3. Die Software zur Erkennung kardiogener Interferenzen nach Anspruch 1, **dadurch gekennzeichnet, dass** sie ferner Folgendes umfasst:
das Bestimmen einer Größe der kardiogenen Interferenz; und
das Anpassen einer Triggerempfindlichkeit entsprechend der Größe der kardiogenen Interferenz.

4. Die Software zur Erkennung kardiogener Interferenzen nach Anspruch 1, **dadurch gekennzeichnet, dass**, wenn das erste Signal das Durchflusssignal umfasst, das zweite Signal ein Drucksignal umfasst;
vorzugsweise spiegelt das Drucksignal eine Einatmungsanstrengung des beatmeten Patienten wider;
vorzugsweise umfasst das Drucksignal, das eine Einatmungsanstrengung des beatmeten Patienten widerspiegelt, mindestens eines der folgenden Signale: ein intrapulmonales Drucksignal, ein Atemwegsdrucksignal, ein Ösophagusdrucksignal, ein transdiaphragmatisches Drucksignal, ein intragastrisches Drucksignal oder ein Carina-Drucksignal.

5. Die Software zur Erkennung kardiogener Interferenzen nach Anspruch 1, **dadurch gekennzeichnet, dass**, wenn das erste Signal das Durchflusssignal umfasst, das zweite Signal ein hämodynamisches Signal umfasst;
vorzugsweise umfasst das hämodynamische Signal mindestens eines der folgenden Signale: ein invasives Blutdrucksignal, ein arterielles Blutdrucksignal, ein zentralvenöses Drucksignal oder ein Pulswellensignal.

6. Die Software zur Erkennung kardiogener Interferenzen nach Anspruch 1, **dadurch gekennzeichnet, dass**, wenn das erste Signal das Durchflusssignal umfasst, das zweite Signal ein Signal des Atmungssystems umfasst, das die Einatmungsanstrengung des beatmeten Patienten widerspiegelt;
vorzugsweise umfasst das Signal des Atmungssystems, das die Einatmungsanstrengung des beatmeten Patienten widerspiegelt, mindestens eines der folgenden Signale: ein Lungenultraschallsignal, ein Zwerchfellultraschallsignal, ein Impedanzsignal oder ein elektrisches Zwerchfellsignal.

7. Die Software zur Erkennung kardiogener Interferenzen nach Anspruch 1, **dadurch gekennzeichnet, dass**, wenn das erste Signal das Drucksignal umfasst, das zweite Signal ein Durchflusssignal umfasst.

8. Die Software zur Erkennung kardiogener Interferenzen nach einem der Ansprüche 1 bis 7, **dadurch gekennzeichnet, dass** das Erkennen einer Schwankung des ersten Signals anhand des ersten Signals und des zweiten Signals Folgendes umfasst:
das Erfassen eines Kreuzkorrelationssignals zwischen dem ersten Signal und dem zweiten Signal auf der Grundlage des ersten Signals und des zweiten Signals; und
das Bestimmen eines Auftretungszeitpunkts der Schwankung anhand des Kreuzkorrelationssignals.

9. Die Software zur Erkennung kardiogener Interferenzen nach Anspruch 2, **dadurch gekennzeichnet, dass** das Erkennen der kardiogenen Interferenz in der Schwankung auf der Grundlage der Charakteristik der Schwankung Folgendes umfasst:
das Feststellen, dass es sich bei einer Schwankung um eine kardiogene Interferenz handelt, wenn die Dauer dieser Schwankung innerhalb eines voreingestellten Bereichs liegt; oder
dass das Erkennen der kardiogenen Interferenz in der Schwankung auf der Grundlage des zeitlichen Merkmals der Schwankung Folgendes umfasst:
das Feststellen, dass mehrere Schwankungen die kardiogene Interferenz darstellen, wenn die mehreren Schwankungen einem voreingestellten Rhythmus entsprechen.

10. Die Software zur Erkennung kardiogener Interferenzen nach Anspruch 2, **dadurch gekennzeichnet, dass**, wenn das erste Signal das Durchflusssignal umfasst, das Erfassen der Dauer jeder Schwankung Folgendes umfasst:
das Ermitteln eines geschätzten Durchflusses für den beatmeten Patienten;
das Erfassen eines Schwankungssignals eines Durchflusses entsprechend einer Differenz zwischen einem tatsächlichen Durchfluss im Durchflusssignal und einem entsprechenden geschätzten Durchfluss; und
das Erfassen der Dauer jeder Schwankung gemäß dem Schwankungssignal des Durchflusses.

11. Die Software zur Erkennung kardiogener Interferenzen nach Anspruch 3, **dadurch gekennzeichnet, dass**, wenn das erste Signal das Durchflusssignal umfasst, das Bestimmen einer Größe der kardiogenen Interferenz Folgendes umfasst:
das Ermitteln eines geschätzten Durchflusses für den beatmeten Patienten;
das Erfassen eines Schwankungssignals eines Durchflusses entsprechend einer Differenz zwischen einem tatsächlichen Durchfluss im Durchflusssignal und einem entsprechenden geschätzten Durchfluss; und
das Bestimmen eines Auftretungszeitpunkts der kardiogenen Interferenz und das weitere Bestimmen der Größe der kardiogenen Interferenz gemäß dem Schwankungssignal des Durchflusses, wobei das Schwankungssignal dem Auftretungszeitpunkt der kardiogenen Interferenz entspricht.

12. Die Software zur Erkennung kardiogener Interferenzen nach Anspruch 10 oder 11, **dadurch gekennzeichnet, dass** das Bestimmen eines geschätzten Durchflusses des beatmeten Patienten Folgendes umfasst:
das Bestimmen des geschätzten Durchflusses zum aktuellen Zeitpunkt gemäß einem Atemmechanikmodell, basierend auf einem tatsächlichen Durchfluss zu einem voreingestellten Zeitpunkt vor dem aktuellen Zeitpunkt; oder
das Durchführen einer linearen Anpassung auf der Grundlage eines tatsächlichen Durchflusses innerhalb eines voreingestellten Zeitraums vor einem aktuellen Zeitpunkt, um den geschätzten Durchfluss zum aktuellen Zeitpunkt zu bestimmen.

13. Die Software zur Erkennung kardiogener Interferenzen nach Anspruch 3, **dadurch gekennzeichnet, dass** das Anpassen einer Triggerempfindlichkeit entsprechend der Stärke der kardiogenen Interferenz Folgendes umfasst:
das Erhöhen der Triggerempfindlichkeit gemäß einer voreingestellten Schrittweite, bis die Triggerempfindlichkeit größer oder gleich einem durch die kardiogene Interferenz verursachten Triggersignal ist;
vorzugsweise umfasst das Verfahren ferner das Anpassen der voreingestellten Schrittweite in Echtzeit entsprechend der Stärke der kardiogenen Interferenz.

14. Die Software zur Erkennung kardiogener Interferenzen nach Anspruch 3, **dadurch gekennzeichnet, dass** das Anpassen einer Triggerempfindlichkeit entsprechend der Stärke der kardiogenen Interferenz Folgendes umfasst:
das Anpassen der Triggerempfindlichkeit während eines Beatmungsvorgangs jeweils so, dass sie größer oder gleich der Stärke der kardiogenen Interferenz ist; oder
das Bestimmen eines vorhergesagten Zeitpunkts der nächsten kardiogenen Interferenz entsprechend einem Auftretungszeitpunkt der aktuellen kardiogenen Interferenz und das Anpassen der Triggerempfindlichkeit für einen Zeitpunkt, der nahe am vorhergesagten Zeitpunkt der nächsten kardiogenen Interferenz liegt, so dass sie größer oder gleich der Stärke der aktuellen kardiogenen Störung ist;
vorzugsweise umfasst das Verfahren ferner das Vorhersagen eines Herzschlagzyklus gemäß einem Zeitintervall zwischen mindestens zwei Auftreten von kardiogenen Interferenzen; und
das Ermitteln des vorhergesagten Zeitpunkts der nächsten kardiogenen Interferenz gemäß dem Auftreten der aktuellen kardiogenen Interferenz und dem Herzschlagzyklus.

15. Eine medizinische Beatmungsvorrichtung (600), die Folgendes umfasst:
eine Beatmungseinheit (610), die dazu ausgelegt ist, einen beatmeten Patienten zu beatmen;
eine Messeinheit (620), die so ausgelegt ist, dass sie den beatmeten Patienten während der Beatmung des beatmeten Patienten überwacht, um ein erstes Signal und ein zweites Signal zu erfassen; und
einen Prozessor (630), der mit der Messeinheit (620) verbunden und dazu ausgelegt ist, das erste Signal und das zweite Signal zu erfassen, wobei der Prozessor (630) ferner dazu ausgelegt ist, die Identifizierungssoftware gemäß einem der Ansprüche 1 bis 14 zur Erkennung der kardiogenen Interferenz auszuführen

## Revendications

1. Logiciel d'identification destiné à mettre en œuvre un procédé d'identification d'interférence cardiogène pour un dispositif de ventilation médicale, **caractérisé en ce que**, ledit logiciel comprenant un code destiné à être traité par ledit dispositif de ventilation médicale pour :
acquérir un premier signal par le dispositif de ventilation médicale par surveillance d'un objet ventilé, où le premier signal comprend un signal de débit ou un signal de pression ;
acquérir un second signal par le dispositif de ventilation médicale par surveillance de l'objet ventilé ;
identifier une fluctuation du premier signal selon le premier signal et le second signal ; et
acquérir une caractéristique de fluctuation de la fluctuation, et identifier l'interférence cardiogène dans la fluctuation sur la base de la caractéristique de fluctuation de la fluctuation.

2. Logiciel d'identification d'interférence cardiogène selon la revendication 1, **caractérisé en ce que**, la caractéristique de fluctuation de la fluctuation comprend une caractéristique de temps de la fluctuation ;
de préférence, la caractéristique de temps de la fluctuation comprend au moins l'un d'une durée de chaque fluctuation et d'un rythme de la fluctuation.

3. Logiciel d'identification d'interférence cardiogène selon la revendication 1, **caractérisé en ce qu'**il comprend en outre :
le fait de déterminer une ampleur de l'interférence cardiogène ; et
le fait d'ajuster une sensibilité de déclenchement selon l'ampleur de l'interférence cardiogène.

4. Logiciel d'identification d'interférence cardiogène selon la revendication 1, **caractérisé en ce que**, lorsque le premier signal comprend le signal de débit, le second signal comprend un signal de pression ;
de préférence, le signal de pression reflète un effort inspiratoire de l'objet ventilé ;
de façon davantage préférée, le signal de pression, lequel reflète un effort inspiratoire de l'objet ventilé, comprend au moins l'un d'un signal de pression intrapulmonaire, d'un signal de pression des voies respiratoires, d'un signal de pression œsophagienne, d'un signal de pression transdiaphragmatique, d'un signal de pression intragastrique, et d'un signal de pression de carène.

5. Logiciel d'identification d'interférence cardiogène selon la revendication 1, **caractérisé en ce que**, lorsque le premier signal comprend le signal de débit, le second signal comprend un signal hémodynamique ;
de façon davantage préférée, le signal hémodynamique comprend au moins l'un d'un signal de pression sanguine invasive, d'un signal de pression sanguine artérielle, d'un signal de pression veineuse centrale, et d'un signal d'onde de pulsation.

6. Logiciel d'identification d'interférence cardiogène selon la revendication 1, **caractérisé en ce que**, lorsque le premier signal comprend le signal de débit, le second signal comprend un signal de système respiratoire, lequel reflète un effort inspiratoire de l'objet ventilé ;
de préférence, le signal de système respiratoire, lequel reflète l'effort inspiratoire de l'objet ventilé, comprend au moins l'un d'un signal d'imagerie ultrasonore des poumons, d'un signal d'imagerie ultrasonore du diaphragme, d'un signal d'imagerie d'impédance, et d'un signal électrique du diaphragme.

7. Logiciel d'identification d'interférence cardiogène selon la revendication 1, **caractérisé en ce que**, lorsque le premier signal comprend le signal de pression, le second signal comprend un signal de débit.

8. Logiciel d'identification d'interférence cardiogène selon l'une quelconque des revendications 1 à 7, **caractérisé en ce que**, le fait d'identifier une fluctuation du premier signal selon le premier signal et le second signal comprend :
le fait d'acquérir un signal de corrélation croisée entre le premier signal et le second signal selon le premier signal et le second signal ; et
le fait de déterminer un temps d'occurrence de la fluctuation selon le signal de corrélation croisée.

9. Logiciel d'identification d'interférence cardiogène selon la revendication 2, **caractérisé en ce que** le fait d'identifier l'interférence cardiogène dans la fluctuation sur la base de la caractéristique de fluctuation de la fluctuation comprend :
le fait de déterminer qu'une fluctuation est l'interférence cardiogène lorsqu'une durée de ladite fluctuation est dans une plage prédéfinie ; ou
le fait d'identifier l'interférence cardiogène dans la fluctuation sur la base de la caractéristique de temps de la fluctuation comprend :
le fait de déterminer que de multiples fluctuations sont l'interférence cardiogène, lorsque lesdites multiples fluctuations suivent un rythme prédéfini.

10. Logiciel d'identification d'interférence cardiogène selon la revendication 2, **caractérisé en ce que**, lorsque le premier signal comprend le signal de débit, le fait d'acquérir la durée de chaque fluctuation comprend :
le fait de déterminer un débit estimé de l'objet ventilé ;
le fait d'acquérir un signal de fluctuation d'un débit selon une différence entre un débit réel dans le signal de débit et un débit estimé correspondant ; et
le fait d'acquérir la durée de chaque fluctuation selon le signal de fluctuation du débit.

11. Logiciel d'identification d'interférence cardiogène selon la revendication 3, **caractérisé en ce que**, lorsque le premier signal comprend le signal de débit, le fait de déterminer une ampleur de l'interférence cardiogène comprend :
le fait de déterminer un débit estimé de l'objet ventilé ;
le fait d'acquérir un signal de fluctuation d'un débit selon une différence entre un débit réel dans le signal de débit et un débit estimé correspondant ; et
le fait de déterminer un temps d'occurrence de l'interférence cardiogène, et en outre de déterminer l'ampleur de l'interférence cardiogène selon le signal de fluctuation du débit, lequel signal de fluctuation correspond au temps d'occurrence de l'interférence cardiogène.

12. Logiciel d'identification d'interférence cardiogène selon la revendication 10 ou 11, **caractérisé en ce que** le fait de déterminer un débit estimé de l'objet ventilé comprend :
le fait de déterminer le débit estimé à un instant actuel selon un modèle de mécanique respiratoire, sur la base d'un débit réel à un instant prédéfini avant l'instant actuel ; ou
le fait de réaliser une régression linéaire sur la base d'un débit réel dans une période prédéfinie avant un instant actuel, de sorte à déterminer le débit estimé à l'instant actuel.

13. Logiciel d'identification d'interférence cardiogène selon la revendication 3, **caractérisé en ce que** le fait d'ajuster une sensibilité de déclenchement selon l'ampleur de l'interférence cardiogène comprend :
le fait d'augmenter la sensibilité de déclenchement selon une longueur de pas prédéfinie jusqu'à ce que la sensibilité de déclenchement soit supérieure ou égale à un signal de déclenchement qui est provoqué par l'interférence cardiogène ;
de préférence, le procédé comprend en outre le fait d'ajuster la longueur de pas prédéfinie en temps réel selon l'ampleur de l'interférence cardiogène.

14. Logiciel d'identification d'interférence cardiogène selon la revendication 3, **caractérisé en ce que** le fait d'ajuster une sensibilité de déclenchement selon l'ampleur de l'interférence cardiogène comprend :
le fait d'ajuster respectivement des sensibilités de déclenchement durant un processus de ventilation pour qu'elles soient supérieures ou égales à l'ampleur de l'interférence cardiogène ; ou
le fait de déterminer un temps prédit de prochaine interférence cardiogène selon un temps d'occurrence d'interférence cardiogène actuelle, et d'ajuster la sensibilité de déclenchement à un temps donné, lequel temps est proche du temps prédit de la prochaine interférence cardiogène, pour qu'elle soit supérieure ou égale à l'ampleur de l'interférence cardiogène actuelle ;
de préférence, le procédé comprend en outre le fait de prédire un cycle cardiaque selon un intervalle de temps entre au moins deux occurrences d'interférence cardiogène ; et
le fait d'acquérir le temps prédit de la prochaine interférence cardiogène selon le temps d'occurrence de l'interférence cardiogène actuelle et le cycle cardiaque.

15. Dispositif de ventilation médicale (600), incluant :
une unité de ventilation (610), laquelle est configurée pour fournir une ventilation à un objet ventilé ;
une unité de mesure (620), laquelle est configurée pour surveiller l'objet ventilé durant la ventilation prodiguée à l'objet ventilé, de sorte à acquérir un premier signal et un second signal ; et
un processeur (630), lequel est relié à l'unité de mesure (620) et configuré pour acquérir le premier signal et le second signal, où le processeur (630) est en outre configuré pour exécuter le logiciel d'identification selon l'une quelconque des revendications 1 à 14 afin d'identifier l'interférence cardiogène.
